# EUROPEAN PATENT SPECIFICATION

(11) **EP 3 459 432 B1**
(45) Date of publication and mention of the grant of the patent: **23.08.2023**
(21) Application number: 18192678.3
(22) Date of filing: 05.09.2018
(51) Int. Cl.: A61B 1/12, A61B 1/00, G01M 3/26

(54) **METHOD OF ACHIEVING VALIDATION OF ENDOSCOPES**
VERFAHREN ZUM ERREICHEN DER VALIDIERUNG VON ENDOSKOPEN
PROCÉDÉ D'ATTEINTE DE VALIDATION D'ENDOSCOPES

(30) Priority: 25.09.2017 NL 2019624
(43) Date of publication of application: 27.03.2019
(73) Proprietor: Van Vliet Medical Supply B.V., 1362 JD Almere (NL)
(72) Inventor: HESEL, Ernst Hendrik Anton, 1311 RW Almere (NL)
(74) Representative: van Breda, Jacobus

(56) References cited:
- EP-A1- 3 111 823
- EP-A2- 2 025 282
- WO-A1-2016/205896
- WO-A1-2017/060390
- US-A1- 2007 238 923

## Description

The present invention relates to a method of providing data to assess whether a - commonly medical - instrument comprising channels to be dried, which is used for examining cavities, meets the safety requirements.

The present invention further relates to a drying cabinet in which the method is applied.

In DE 3835861 A1 a description is given of a drying cabinet in which the interior and the exterior of endoscopes is blow-dried using increased air pressure pulses.

In EP 1779767 A1 a description is given of a processor by means of which the operation of endoscopes can be validated.

US 5,840,251 describes inter alia the drying process of interior channels of an endoscope.

WO 2005/056060 describes inter alia the preservation of endoscopes by injecting hot air into the channels thereof.

WO 2016/046503 describes the drying of endoscopes by sending a neutral gas, at low flow rates, during specific periods and within specific temperature ranges, through endoscope channels.

The information from these documents does not make it possible to achieve professional validation of the degree of dryness of interior channels in such instruments in a reliable and verifiable manner.

It is an object of the present invention to provide an improved and more widely applicable method and drying cabinet for drying and, if necessary, subsequent validation of, in particular, endoscopes, providing reliable data with respect to the various types of defects which may affect the interior channels of such an instrument as well as with respect to which one of the channels is affected.

A method according to the preamble of claim 1, and an apparatus according to the preamble of claim 9 are known from both US2007/0238923 and from EP 3 111 823.

In US2007/0238923 an endoscope integrity tester is disclosed having on top a compartment which can receive an endoscope to be tested.

EP 3 111 823 discloses a leak tester for an endoscope, wherein the endoscope is wound in an annular shape.

WO2016/205896 discloses a sanitary monitoring system, the system including: one or more conduits configured to be connected to a medical device; one or more measurement devices configured to assist in determining one or more flow rates associated with the medical device from a fluid flow provided through the one or more conduits; and a status device configured to determine a sanitary condition of the medical device from the one or more flow rates.

According to the invention a method and apparatus are proposed in accordance with claims 1 and 9, respectively.

An advantage of the method according to the invention resides in that the distribution means connected to the proximal ends of the interior channels in the instrument, enable the supplied, sterile compressed air to be evenly distributed among the connected channels. If a channel is partly or fully clogged, then such a distribution is either not possible or the measured amount of incoming compressed air deviates from the usual amount for the instrument under examination, which is a direct indication that one or more channels are clogged, or exhibit mutual leakage or leakage to the exterior. These defects may cause infections.

The method according to the invention further has the advantage that if the measuring devices are connected to one or more input or output channel ends, it is possible, based on individual measurement per channel of, for example, in- or outgoing air flow rates, temperatures and/or moisture content of the air, to determine from the measured deviations or differences which one of the measured channels is clogged, and to what degree, or shows signs of leakage.

Consequently, in the case of repair it is possible, in principle, to deliberately close the defective channel completely, so that, for example, endoscopes costing many tens of thousands of Euros, are not rendered completely unusable. Endoscopes comprise, for example, at least a suction channel, a water channel, an air channel, a biopsy channel, an auxiliary channel and frequently also one or more spare channels which function properly and hence can be used instead of the defective, closed channel, thereby enabling normal use of the endoscope.

An additional advantage of the method according to the invention resides in that by measuring the temperature and/or moisture content at least at the distal channel ends, it is possible to determine the degree to which the instrument is dry and safe for use. In general, the moisture content has decreased to such extent after a few hours that measurements show that the instrument is dry. It can furthermore be stated that if the moisture content, i.e., in general the absolute or relative moisture content, of the outgoing compressed air no longer changes or is lower than a specific level, the channels no longer contain any moisture, which can be demonstrated through measurements; this is in itself a reliable and verifiable measure of the dry state and operational safety of the instrument.

If a specific protocol is adopted and the measurements are carried out as explained hereinabove, the above-mentioned air parameter quantities measured by the measuring devices enable an official validation of instruments to be based thereon, in particular endoscopes, leading to safe, reliable and verifiable use in hospitals and research centres.

Further detailed, possible embodiments explained in the other claims are mentioned, together with the associated advantages, in the following description.

The method and the drying cabinet according to the invention for the intended instruments will now be explained in greater detail with reference to the figures mentioned below, in which corresponding elements are indicated by means of the same reference numerals.
In the figures:
Figure 1 is a cross-sectional view of an endoscope by means of which the method according to the invention will be explained; and
Figure 2 shows a drying cabinet according to the invention in which endoscopes are suspended which are subjected to the method according to the invention.

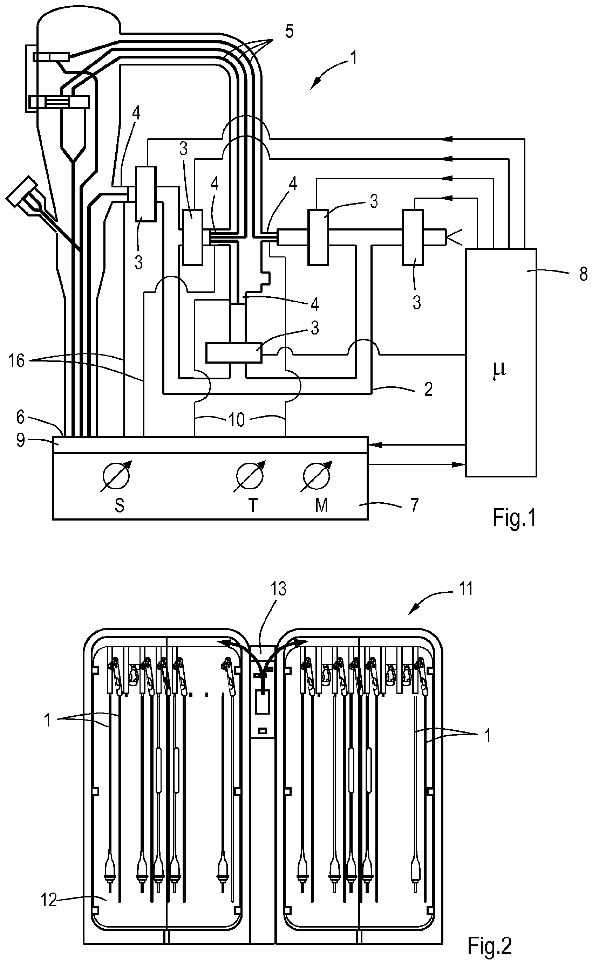

Figure 1 is a partly cross-sectional view of an endoscope 1. For the purpose of this document, the term "endoscope" includes any instrument capable of treating and/or examining cavities in human beings and/or animals. From the right-hand side in figure 1, air, preferably sterile compressed air because it is sufficiently free of bacterial, viral contamination and/or dust particles, is supplied to a system of air pipes 2. Depending on the requirements, the compressed air can be supplied in a continuous or pulsating manner. Said branched system 2 extends via air-distribution means 3 to adapters, not shown explicitly, which ensure connection to proximal channel ends 4 of various channels 5 extending in the endoscope 1, which channels need to be dried after the endoscope has been cleaned. Examples of channels 5 include: a suction channel, a water channel, an air channel, a biopsy channel or one or more auxiliary or spare channels.

If the channels 5 are different in cross-section, the air flows through the channels at different velocities, as a result of which, a smaller channel through which less air flows, is dried insufficiently or not at all, which may lead to contamination with, for example, pathogens. To preclude this, the air distribution means 3 comprise fixed or adjustable air restrictions. Fixed restrictions, in the sense that the relevant actual air resistances cannot be influenced, can be used if endoscopes of one known type are dried, whose channel length and the individual cross-sections of the channels are known before the start of the drying process. In this case, said fixed restrictions may be adapted to said known channel data, so that uniform and, if necessary, laminar flows of drying air with known flow rate through each one of the channels 5 can be generated. Adjustable air restrictions are used with endoscopes of different types having different channel diameters not known in advance. In the latter case, the air restrictions can be adjusted and are set such that desired air flows or air volumes pass through the channels 5. To achieve this, the outgoing air flow rates at distal channel ends 6 are determined for each channel by connected measuring instruments 7 and these data are supplied to an electronic control unit 8 which is connected to each one of the respective adjustable restrictions in the distribution means 3 to enable air flow control for each channel individually.

In general, measuring instruments 7 can be connected to the distal channel ends 6 to measure one or more of the outgoing air parameters, such as flow rate S, temperature T and/or moisture content M. If an endoscope 1 has been cleaned and is offered for drying and, at a later point in time, validation, it may be that the above-explained flow of air through a specific channel does not take place within the customary adjustment range of the restrictions. This indicates a problem with said channel. In the case of a defective channel, it is generally necessary to replace the endoscope, because a defective channel poses a safety risk, inter alia, a contamination risk.

The measuring instruments 7 can be used for measuring in various ways. For all channels, each one of the incoming and/or outgoing air parameters deemed relevant can be measured continuously, as a result of which this method is costly. Alternatively, other measuring instruments 7 can be used to (also) continuously and simultaneously measure all incoming and/or outgoing air parameters deemed relevant.

If change-over switches 9, as diagrammatically shown in figure 1, are used for switching one or more measuring instruments 7 successively from one proximal and/or distal channel end 4, 6, respectively, to another, then one set of measuring instruments 7 is sufficient to measure sequentially the respective air parameters in all channels 5, as a result of which this way of measuring is less costly. Figure 1 also shows the possibility to measure relevant incoming air parameters, such as the flow rate of the incoming air, at the input channel ends 4 by using only one set of instruments 7. In this case, the air pipes 10, 16 shown are situated between the input channel ends 4 and the change-over switches 9. The control unit 8 comprising a computing element can be used, if required, to compare the incoming and outgoing parameters, against each other if necessary, and act upon the outcome by setting the adjustable air restrictions.

Figure 2 shows a drying cabinet 11 comprising one, or as shown in the figure, two drying spaces 12 which are arranged such that one or more instruments 1 can be suspended therein. The proximal ends 4 of the channels 5 in the endoscopes 1 are suspended at the top of the drying spaces 12, wherein a specific excess pressure is produced, so that air contaminated by external sources of infection is prevented from entering. The air distribution means 3, not shown here, can be connected to the proximal channel ends 4, if necessary together with the relevant measuring instruments 7, by means of the partly flexible system 2, and, likewise, the distal ends 6 at the bottom of the drying space(s) 12 can be connected to the measuring instruments 7, not shown here, to measure the air parameters, such as flow rate, temperature and/or moisture content of incoming and outgoing air, respectively. The moisture content is measured at the channel ends 4, 6 by determining the relative and/or absolute air humidity at said locations in known manner. By means of the previously explained change-over switches 9, the incoming/outgoing air parameters can be advantageously measured by means of the same measuring instruments 7. A technical compartment 13 is situated at the side of drying cabinets 11, in this case between drying cabinets, and is intended also to produce air flows which pass through the spaces 12, from top to bottom, and along the exterior of the freely suspended endoscopes 1 to be dried also. The front of the spaces 12 is provided with doors having coloured illumination at the top part thereof and secured closures between the endoscopes 1 and their suspension points, which are released only after an authorized user has registered. If the measurements show that the parameters range within the required limiting values, the instrument can be qualified as safe for use and validated.

## Claims

1. A method of providing data to assess whether a - commonly medical - instrument (1) comprising interior channels (5) to be dried, which is used for examining cavities, meets the safety requirements, wherein sterile compressed air is led through air distribution means (3) to respective channel ends, and measuring devices (7) are connected to one or more of the respective proximal and/or distal channel ends for measuring the flow rate, temperature and/or moisture content of the incoming and/or outgoing air at the channel ends, the method providing a drying cabinet (11) with one or more drying spaces (12), and freely suspending in each drying space a plurality of instruments (1), such that proximal ends of the channels (5) in the instruments (1) are suspended at the top of the drying space (12), where the drying cabinet (11) is provided with the air distribution means (3), connected to compressed air, and connecting the measuring instruments (7) to the proximal channel ends, and connecting distal channel ends of the relevant suspended instruments (1) at the bottom of the drying space (12) to the measuring instruments (7) provided in the drying cabinet (11), and using the abovementioned measuring instruments (7) to measure flow rate, temperature and/or moisture content of the incoming and/or outgoing air, and
**characterised in that** the drying cabinet (11) is arranged such that an air excess pressure can be maintained in the one or more drying spaces (12) thereof to prevent infection, pathogens or dust from entering the instruments present in said spaces.

2. The method according to claim 1, **characterized in that** the compressed air is supplied to said proximal channel ends in a continuous or pulsating flow.

3. The method according to claim 1 or 2, **characterized in that** the measuring instruments (7) are used to measure, either continuously or sequentially, the flow rate, temperature and/or moisture content at at least one of the channel ends.

4. The method according to claim 3, **characterized in that** after the said measurement, the measuring instruments (7) are changed over to a subsequent channel end to carry out a next measurement.

5. The method according to any one of the claims 1 to 4, **characterized in that** with respect to flow rate, temperature and/or moisture content of one or more of these air parameters, the difference is measured between the air entering and leaving the respective channel end.

6. The method according to claim 5, **characterized in that** the difference(s) per channel (5) are measured continuously or sequentially.

7. The method according to claim 6, **characterized in that** the said difference is used to determine whether a channel (5) leaks or is clogged.

8. The method according to any one of claims 1 to 7, **characterized in that** the air distribution means (3) comprise fixed or adjustable distribution means (3), the latter means being arranged such that they comprise adjustable air restrictions which are set in such a manner that the air flow is uniformly distributed among the respective channels and/or is a laminar air flow in said channels.

9. A drying cabinet wherein the method according to any one of claims 1 to 8 is applied to dry, using sterile compressed air, interior channels in an instrument (1), such as an endoscope, bronchoscope, colonoscope, sigmoidoscope or other instrument comprising interior channels (5) for examination of mostly human or animal cavities, wherein the drying cabinet (11) comprises one or more drying spaces (12), and each drying space (12) is arranged such that a plurality of instruments (1) can be freely suspended therein, and that proximal ends of the channels (5) in the instruments (1) are suspended at the top of the drying space (12), where the drying cabinet (11) is provided with air distribution means (3), connected to compressed air, and measuring instruments (7) to which the proximal channel ends can be connected, and distal channel ends of the relevant suspended instruments (1) can be connected at the bottom of the drying space to measuring instruments (7) provided in the drying cabinet (11), enabling the abovementioned measuring instruments (7) to measure flow rate, temperature and/or moisture content of the incoming and/or outgoing air,
**characterised in that** the drying cabinet (11) is arranged such that an air excess pressure can be maintained in the one or more drying spaces (12) thereof to prevent infection, pathogens or dust from entering the instruments present in said spaces.

10. The drying cabinet according to claim 9, **characterized in that** the cabinet is provided with a technical compartment 13 for producing air flows which pass through the drying spaces (12), from top to bottom, and along the exterior of the freely suspended endoscopes (1) to be dried also.

11. The drying cabinet according to claim 9 or 10, **characterized in that** the drying cabinet (11) is provided with change-over means (9) which are connected, on the one hand, to the proximal and/or distal channel ends and, on the other hand, to the measuring instruments (7) in order to be able to measure flow rate, temperature and/or moisture content in the proximal and/or distal channel ends by means of the same measuring instruments (7).

12. The drying cabinet according to any one of claims 9 to 11, **characterized in that** the air distribution means (3) comprise fixed or adjustable air restrictions which can be set by means of an electronic control unit (8) which cooperates with the drying cabinet (11) in order to be able to influence individual, incoming air flows to one or more input channel ends.

13. The drying cabinet according to claim 12, **characterized in that** the control unit (8) receives information regarding the flow rate of the air leaving the distal ends from the abovementioned measuring instruments (7), which flow rates are compared and subsequently the air restrictions are adjusted by means of the control unit (8), such that a uniform and/or laminar flow of air through each channel (5) is generated.

14. The drying cabinet according to any one of claims 9 to 13, **characterized in that** the channels (5) in the instruments (1) form one or more of the following channels: a suction channel, a water channel, an air channel, a biopsy channel or a spare/auxiliary channel.

## Patentansprüche

1. Ein Verfahren zur Bereitstellung von Daten zur Beurteilung, ob ein - üblicherweise medizinisches - Instrument (1) mit zu trocknenden Innenkanälen (5), das zur Untersuchung von Hohlräumen verwendet wird, den Sicherheitsanforderungen entspricht, wobei sterile Druckluft durch Luftverteilungsmittel (3) zu den jeweiligen Kanalenden geleitet wird und Messinstrumente (7) mit einem oder mehreren der jeweiligen proximalen und/oder distalen Kanalenden verbunden sind, um die Durchflussrate, die Temperatur und/oder den Feuchtigkeitsgehalt der ein- und/oder ausströmenden Luft an den Kanalenden zu messen, das Verfahren einen Trocknungsschranks (11) mit einem oder mehreren Trocknungsräumen (12) bereitstellt und frei hängend in jedem Trocknungsraum eine Vielzahl von Instrumenten (1), so dass die proximalen Enden der Kanäle (5) in den Instrumenten (1) an der Oberseite des Trocknungsraums (12) aufgehängt sind, wobei der Trocknungsschrank (11) mit Luftverteilungsmitteln (3) versehen ist, die an Druckluft angeschlossen sind, und die Messinstrumente (7) mit den proximalen Kanalenden verbindet und die distalen Kanalenden der betreffenden aufgehängten Instrumente (1) am Boden des Trocknungsraums (12) mit den im Trocknungsschrank (11) vorgesehenen Messinstrumenten (7) verbindet und die vorgenannten Messinstrumente (7) zum Messen der Strömungsgeschwindigkeit, der Temperatur und/oder des Feuchtigkeitsgehalts der ein- und/oder ausströmenden Luft verwendet, und
**dadurch gekennzeichnet, dass**
der Trockenschrank (11) so ausgestaltet ist, dass in einem oder mehreren Trockenräumen (12) ein Luftüberdruck aufrechterhalten werden kann, um zu verhindern, dass Infektionen, Krankheitserreger oder Staub in die in den Räumen befindlichen Instrumente gelangen.

2. Das Verfahren nach Anspruch 1,
**dadurch gekennzeichnet, dass**
die Druckluft den proximalen Kanalenden in einem kontinuierlichen oder pulsierenden Strom zugeführt wird.

3. Das Verfahren nach Anspruch 1 oder 2,
**dadurch gekennzeichnet, dass**
die Messinstrumente (7) zur kontinuierlichen oder sequentiellen Messung der Durchflussmenge, der Temperatur und/oder des Feuchtigkeitsgehalts an mindestens einem der Kanalenden verwendet werden.

4. Das Verfahren nach Anspruch 3,
**dadurch gekennzeichnet, dass**
nach besagter Messung die Messinstrumente (7) auf ein nachfolgendes Kanalende gewechselt werden, um eine weitere Messung durchzuführen.

5. Das Verfahren nach einem der Ansprüche 1 bis 4,
**dadurch gekennzeichnet, dass**
in Bezug auf die Durchflussmenge, die Temperatur und/oder den Feuchtigkeitsgehalt eines oder mehrerer dieser Luftparameter die Differenz zwischen der in das jeweilige Kanalende einströmenden und der aus ihm ausströmenden Luft gemessen wird.

6. Das Verfahren nach Anspruch 5,
**dadurch gekennzeichnet, dass**
die Differenz(en) pro Kanal (5) kontinuierlich oder sequentiell gemessen werden.

7. Das Verfahren nach Anspruch 6,
**dadurch gekennzeichnet, dass**
anhand dieser Differenz festgestellt wird, ob ein Kanal (5) undicht oder verstopft ist.

8. Das Verfahren nach einem der Ansprüche 1 bis 7,
**dadurch gekennzeichnet, dass**
die Luftverteilungsmittel (3) feste oder einstellbare Verteilungsmittel (3) umfassen, wobei die letzteren Mittel so ausgestaltet sind, dass sie einstellbare Luftbegrenzungen umfassen, die so eingestellt sind, dass der Luftstrom gleichmäßig auf die jeweiligen Kanäle verteilt wird und/oder ein laminarer Luftstrom in den Kanälen ist.

9. Ein Trockenschrank, in dem das Verfahren nach einem der Ansprüche 1 bis 8 angewandt wird, um unter Verwendung von steriler Druckluft Innenkanäle in einem Instrument (1), wie beispielsweise einem Endoskop, Bronchoskop, Kolonoskop, Sigmoidoskop oder einem anderen Instrument mit Innenkanälen (5) zur Untersuchung von hauptsächlich menschlichen oder tierischen Hohlräumen, zu trocknen, wobei
der Trocknungsschrank (11) einen oder mehrere Trocknungsräume (12) umfasst, und jeder Trocknungsraum (12) so angeordnet ist, dass eine Vielzahl von Instrumenten (1) darin frei aufgehängt werden kann, und dass proximale Enden der Kanäle (5) in den Instrumenten (1) an der Oberseite des Trocknungsraums (12) aufgehängt sind, wobei der Trocknungsschrank (11) mit Luftverteilungsmitteln (3) versehen ist, die mit Druckluft verbunden sind, und Messinstrumenten (7) versehen ist, an die die proximalen Kanalenden angeschlossen werden können, und die distalen Kanalenden der betreffenden aufgehängten Instrumente (1) am Boden des Trocknungsraums mit Messinstrumenten (7) verbunden werden können, die in dem Trocknungsschrank (11) vorgesehen sind, wodurch die vorgenannten Messinstrumente (7) in die Lage versetzt werden, die Durchflussmenge, die Temperatur und/oder den Feuchtigkeitsgehalt der ein- und/oder ausströmenden Luft zu messen,
**dadurch gekennzeichnet, dass**
der Trockenschrank (11) so ausgestaltet ist, dass in einem oder mehreren Trockenräumen (12) ein Luftüberdruck aufrechterhalten werden kann, um zu verhindern, dass Infektionen, Krankheitserreger oder Staub in die in den Räumen befindlichen Instrumente gelangen.

10. Der Trockenschrank nach Anspruch 9,
**dadurch gekennzeichnet, dass**
der Schrank mit einem Technikraum (13) zur Erzeugung von Luftströmen versehen ist, welche die Trocknungsräume (12) von oben nach unten und entlang der Außenseite der zu trocknenden, frei hängenden Endoskope (1) durchströmen.

11. Der Trockenschrank nach Anspruch 9 oder 10,
**dadurch gekennzeichnet, dass**
der Trockenschrank (11) mit Umschaltmitteln (9) versehen ist, die einerseits mit den proximalen und/oder distalen Kanalenden und andererseits mit den Messinstrumenten (7) verbunden sind, um Durchfluss, Temperatur und/oder Feuchtigkeitsgehalt in den proximalen und/oder distalen Kanalenden mittels derselben Messinstrumente (7) messen zu können.

12. Trockenschrank nach einem der Ansprüche 9 bis 11,
**dadurch gekennzeichnet, dass**
die Luftverteilungsmittel (3) feste oder einstellbare Luftbegrenzungen umfassen, die mittels einer elektronischen Steuereinheit (8), die mit dem Trockenschrank (11) zusammenwirkt, eingestellt werden können, um einzelne, eintretende Luftströme zu einem oder mehreren Eingangskanalenden beeinflussen zu können.

13. Der Trockenschrank nach Anspruch 12,
**dadurch gekennzeichnet, dass**
die Steuereinheit (8) von den oben genannten Messinstrumenten (7) Informationen über die Strömungsgeschwindigkeit der aus den distalen Enden ausströmenden Luft erhält, wobei diese Strömungsgeschwindigkeiten verglichen werden und anschließend die Luftbegrenzungen mittels der Steuereinheit (8) so eingestellt werden, dass eine gleichmäßige und/oder laminare Luftströmung durch jeden Kanal (5) erzeugt wird.

14. Der Trockenschrank nach einem der Ansprüche 9 bis 13,
**dadurch gekennzeichnet, dass**
die Kanäle (5) in den Instrumenten (1) einen oder mehrere der folgenden Kanäle bilden: einen Saugkanal, einen Wasserkanal, einen Luftkanal, einen Biopsiekanal oder einen Reserve-/Hilfskanal.

## Revendications

1. Procédé de fourniture de données pour évaluer si un instrument (1), généralement médical, comprenant des canaux intérieurs (5) à sécher, qui est utilisé pour examiner des cavités, satisfait aux exigences de sécurité, dans lequel de l'air comprimé stérile est conduit à travers des moyens de distribution d'air (3) jusqu'à des extrémités respectives de canal, et des dispositifs de mesure (7) sont reliés à une ou plusieurs des extrémités de canal proximales et/ou distales respectives pour mesurer les débit, température et/ou teneur en humidité de l'air entrant et/ou sortant au niveau des extrémités de canal, le procédé fournissant une enceinte de séchage (11) ayant un ou plusieurs espaces de séchage (12), et suspendant librement dans chaque espace de séchage une pluralité d'instruments (1), de sorte que des extrémités proximales des canaux (5) dans les instruments (1) sont suspendues au niveau du sommet de l'espace de séchage (12), où l'enceinte de séchage (11) est munie des moyens de distribution d'air (3), reliés à de l'air comprimé, et reliant les instruments de mesure (7) aux extrémités proximales de canal, et reliant des extrémités distales de canal des instruments suspendus concernés (1) au niveau du fond de l'espace de séchage (12) aux instruments de mesure (7) disposés dans l'enceinte de séchage (11), et utilisant les instruments de mesure (7) mentionnés ci-dessus pour mesurer les débit, température et/ou teneur en humidité de l'air entrant et/ou sortant, et
caractérisé en que l'enceinte de séchage (11) est agencée de telle sorte qu'une surpression d'air peut être maintenue dans les un ou plusieurs espaces de séchage (12) de celle-ci pour éviter qu'une contamination, des agents pathogènes ou de la poussière ne pénètrent dans les instruments présents dans lesdits espaces.

2. Procédé selon la revendication 1, **caractérisé en ce que** l'air comprimé est fourni auxdites extrémités proximales de canal en un flux continu ou pulsé.

3. Procédé selon la revendication 1 ou 2, **caractérisé en ce que** les instruments de mesure (7) sont utilisés pour mesurer, de manière continue ou séquentielle, les débit, température et/ou teneur en humidité au niveau d'au moins une des extrémités de canal.

4. Procédé selon la revendication 3, **caractérisé en ce qu'**après ladite mesure, les instruments de mesure (7) sont permutés vers une extrémité de canal suivante pour effectuer une mesure suivante.

5. Procédé selon l'une quelconque des revendications 1 à 4, **caractérisé en ce que**, en ce qui concerne les débit, température et/ou teneur en humidité parmi un ou plusieurs de ces paramètres d'air, la différence est mesurée entre l'air entrant dans l'extrémité de canal respective et quittant celle-ci.

6. Procédé selon la revendication 5, **caractérisé en ce que** la ou les différences par canal (5) sont mesurées de manière continue ou séquentielle.

7. Procédé selon la revendication 6, **caractérisé en ce que** ladite différence est utilisée pour déterminer si un canal (5) fuit ou est bouché.

8. Procédé selon l'une quelconque des revendications 1 à 7, **caractérisé en ce que** les moyens de distribution d'air (3) comprennent des moyens de distribution fixes ou réglables (3), ces derniers moyens étant agencés de telle sorte qu'ils comprennent des restrictions d'air réglables qui sont établies de manière à ce que le flux d'air soit uniformément réparti entre les canaux respectifs et/ou soit un flux d'air laminaire dans lesdits canaux.

9. Enceinte de séchage dans laquelle le procédé selon l'une quelconque des revendications 1 à 8 est appliqué pour sécher, à l'aide d'air comprimé stérile, des canaux intérieurs dans un instrument (1), tel qu'un endoscope, un bronchoscope, un colonoscope, un sigmoïdoscope ou un autre instrument comprenant des canaux intérieurs (5) pour examiner des cavités principalement d'êtres humains ou d'animaux, dans laquelle l'enceinte de séchage (11) comprend un ou plusieurs espaces de séchage (12), et chaque espace de séchage (12) est agencé de sorte qu'une pluralité d'instruments (1) peuvent être librement suspendus à l'intérieur, et que des extrémités proximales des canaux (5) dans les instruments (1) sont suspendues au sommet de l'espace de séchage (12), où l'enceinte de séchage (11) est munie de moyens de distribution d'air (3), reliés à de l'air comprimé, et des instruments de mesure (7) auxquels les extrémités proximales de canal peuvent être reliées, et des extrémités distales de canal des instruments suspendus concernés (1) peuvent être reliées au niveau du fond de l'espace de séchage à des instruments de mesure (7) disposés dans l'enceinte de séchage (11), permettant aux instruments de mesure (7) mentionnés ci-dessus de mesurer les débit, température et/ou teneur en humidité de l'air entrant et/ou sortant,
**caractérisée en ce que** l'enceinte de séchage (11) est agencée de telle sorte qu'une surpression d'air peut être maintenue dans les un ou plusieurs espaces de séchage (12) de celle-ci pour éviter qu'une contamination, des agents pathogènes ou de la poussière ne pénètrent dans les instruments présents dans lesdits espaces.

10. Enceinte de séchage selon la revendication 9, **caractérisée en ce que** l'enceinte est munie d'un compartiment technique (13) pour produire des flux d'air qui traversent les espaces de séchage (12), de haut en bas, et le long de l'extérieur des endoscopes librement suspendus (1) à sécher également.

11. Enceinte de séchage selon la revendication 9 ou 10, **caractérisée en ce que** l'enceinte de séchage (11) est munie de moyens de permutation (9) qui sont reliés, d'une part, aux extrémités de canal proximales et/ou distales et, d'autre part, aux instruments de mesure (7) afin de pouvoir mesurer les débit, température et/ou teneur en humidité dans les extrémités de canal proximales et/ou distales au moyen des mêmes instruments de mesure (7).

12. Enceinte de séchage selon l'une quelconque des revendications 9 à 11, **caractérisée en ce que** les moyens de distribution d'air (3) comportent des restrictions d'air fixes ou réglables qui peuvent être établies au moyen d'une unité de commande électronique (8) qui coopère avec l'enceinte de séchage (11) afin de pouvoir influencer des flux d'air entrant individuels vers une ou plusieurs extrémités de canal d'entrée.

13. Enceinte de séchage selon la revendication 12, **caractérisée en ce que** l'unité de commande (8) reçoit des informations concernant le débit d'air quittant les extrémités distales provenant des instruments de mesure (7) mentionnés ci-dessus, lesquels débits sont comparés et ensuite les restrictions d'air sont réglées au moyen de l'unité de commande (8), de sorte qu'un flux d'air uniforme et/ou laminaire à travers chaque canal (5) est généré.

14. Enceinte de séchage selon l'une quelconque des revendications 9 à 13, **caractérisée en ce que** les canaux (5) dans les instruments (1) forment un ou plusieurs des canaux suivants : un canal d'aspiration, un canal d'eau, un canal d'air, un canal de biopsie ou un canal de rechange/auxiliaire.
